# EUROPÄISCHE PATENTSCHRIFT

(11) **EP 1 015 583 B1**
(45) Veröffentlichungstag und Bekanntmachung des Hinweises auf die Patenterteilung: **19.10.2005**
(21) Anmeldenummer: 98905246.9
(22) Anmeldetag: 09.01.1998
(51) Int. Cl.: C12N 15/12, C12N 15/70, C12N 1/21, C12Q 1/68, C07K 14/47, C07K 16/18, A61K 38/17, A61K 48/00, G01N 33/50

(54) **SRCR DOMÄNE-ENTHALTENDES PROTEIN**
PROTEIN CONTAINING AN SRCR DOMAIN
PROTEINE CONTENANT UN DOMAINE SRCR

(30) Priorität: 09.01.1997 DE 19700519; 18.07.1997 DE 19730997
(43) Veröffentlichungstag der Anmeldung: 05.07.2000
(73) Patentinhaber: Deutsches Krebsforschungszentrum Stiftung des öffentlichen Rechts, 69120 Heidelberg (DE)
(72) Erfinder: MOLLENHAUER, Jan, D-69250 Schönau (DE); POUSTKA, Annemarie, D-69120 Heidelberg (DE)
(74) Vertreter: Schüssler, Andrea
(86) Internationale Anmeldenummer: PCT/DE1998/000096
(87) Internationale Veröffentlichungsnummer: WO 1998/030687

(56) Entgegenhaltungen:
- HILLIER L. ET AL.: "The WashU-Merck EST Project, AC T89953" EMBL DATABASE, 7.April 1995, HEIDELBERG, XP002069500
- RESNICK D. ET AL.: "The SRCR superfamily: a family reminiscent of the Ig superfamily" TRENDS IN BIOCHEMICAL SCIENCES, Bd. 19, Nr. 1, Januar 1994, Seiten 5-8, XP002069501
- RASHEED B. ET AL.: "Chromosome 10 deletion mapping in human gliomas: a common deletion region in 10q25" ONCOGENE, Bd. 10, Nr. 11, 1.Juni 1995, Seiten 2243-2246, XP002069757
- ALBAROSA R. ET AL.: "Deletion mapping of gliomas suggests the presence of two small regions for candidate tumor-suppressor genes in a 17-cm interval on chromosome 10q" AMERICAN JOURNAL OF HUMAN GENETICS, Bd. 58, Nr. 6, Juni 1996, Seiten 1260-1267, XP002069758
- MOLLENHAUER J. ET AL.: "DMBT1, a new member of the SRCR superfamily, on chromosome 10q25.3-26.1 is deleted in malignant brain tumours" NATURE GENETICS, Bd. 17, Nr. 1, September 1997, Seiten 32-39, XP002069502

## Beschreibung

Die vorliegende Erfindung betrifft ein eine SRCR Domäne-enthaltendes Protein, eine ein solches kodierende Nukleinsäure und ein Verfahren zur Herstellung eines solchen. Ferner betrifft die Erfindung die Verwendung der Nukleinsäure und des Proteins sowie gegen das Protein gerichtete Antikörper.

Der Ausdruck "SRCR" Domäne bedeutet "scavenger receptor cysteine rich" Domäne. Eine solche Domäne umfaßt etwa 110 Aminosäuren und findet sich in vielen Proteinen, die mit elementaren Prozessen der Zelle, z.B. Zelldifferenzierung oder Zell-Zell-Kontakt, zu tun haben. Dennoch sind diese Prozesse im Detail bisher nicht verstanden.

Der vorliegenden Erfindung liegt somit die Aufgabe zugrunde, ein Mittel bereitzustellen, mit dem man elementare Prozesse der Zelle untersuchen und ggfs. in sie eingreifen kann.

Erfindungsgemäß wird dies durch die Gegenstände in den Patentansprüchen erreicht.

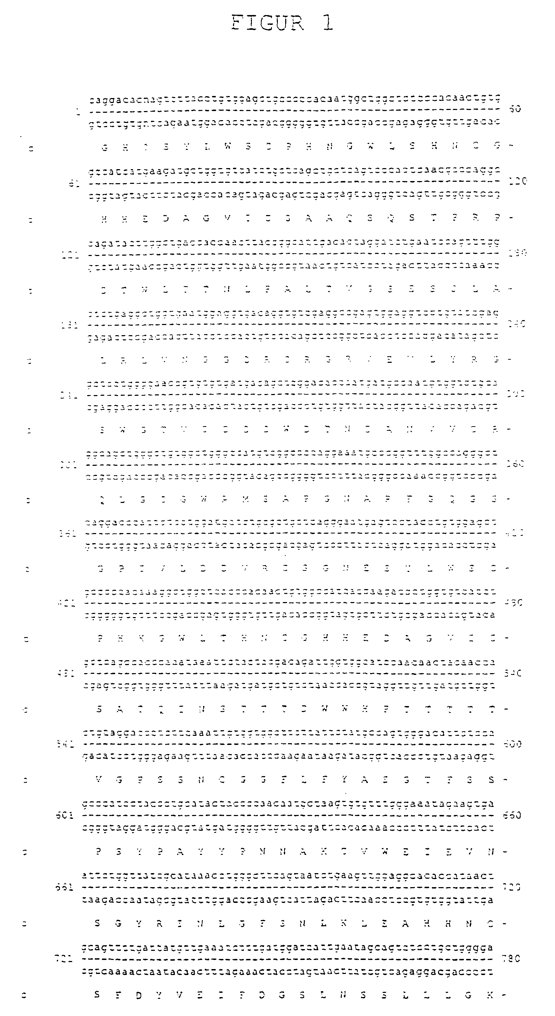

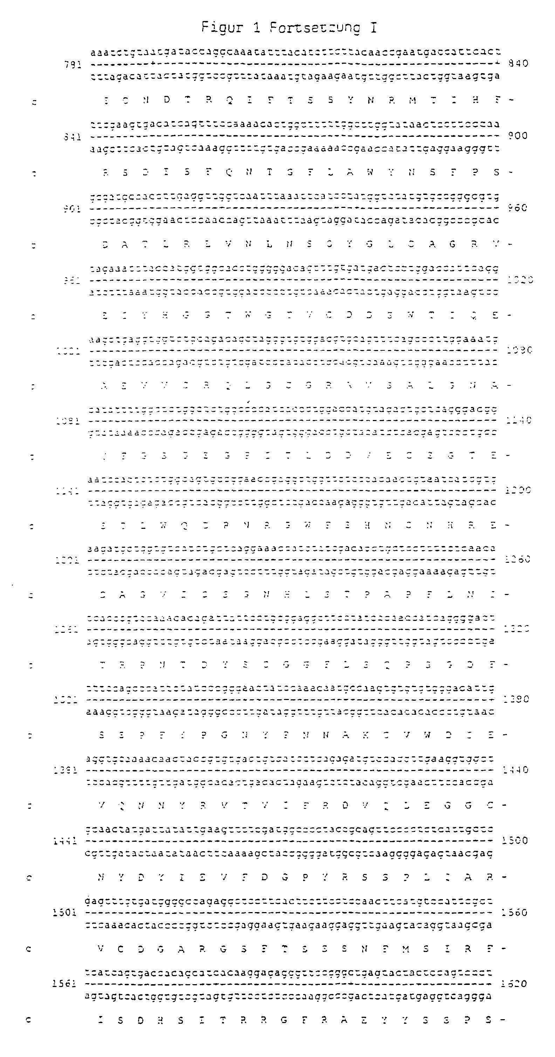

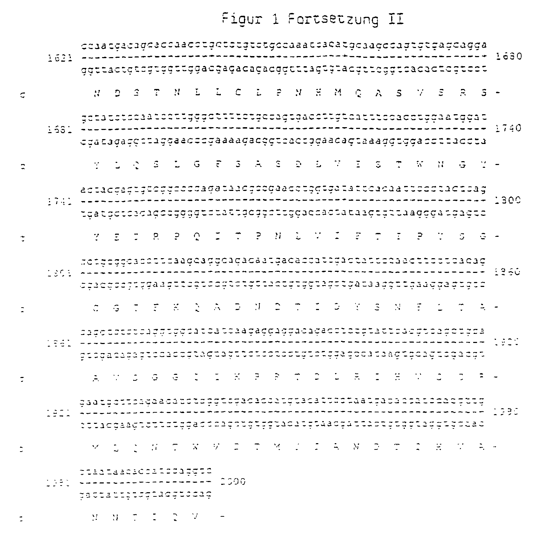

Gegenstand der vorliegenden Erfindung ist somit ein eine SRCR Domäne-enthaltendes Protein, wobei das Protein die Aminosäuresequenz von Fig. 1 oder eine hiervon durch eine oder mehrere Aminosäuren unterschiedliche Aminosäuresequenz umfaßt.

Die vorliegende Erfindung beruht auf der Erkenntnis des Anmelders, daß in Tieren, besonders Säugetieren, ganz besonders dem Menschen, ein eine SRCR Domäne-enthaltendes Protein existiert, das Homologien zu bekannten eine SRCR Domäne-enthaltenden Proteinen aufweist, sich aber von diesen Proteinen auf dem DNA-Level durch Hybridisierung unter üblichen Bedingungen unterscheidet. Ein solches Protein umfaßt die Aminosäuresequenz von Fig. 1 oder eine hiervon durch eine oder mehrere Aminosäuren unterschiedliche Aminosäuresequenz. Ferner hat der Anmelder erkannt, daß das Protein auch eine CUB Domäne enthält, die ebenfalls etwa 110 Aminosäuren umfaßt. Desweiteren hat er gefunden, daß das Protein in Tumorzellen in anderer Form als in Normalzellen vorliegen kann. Die veränderte Form kann sich in Additionen, Substitutionen, Inversionen und/oder Deletionen von einer oder mehreren Aminosäuren darstellen. Insbesondere hat der Anmelder gefunden, daß in Medulloblastomen und Glioblastomen sowie bei Brustkrebs das Protein eine Deletion von einer oder mehreren Aminosäuren aufweisen kann.

In der vorliegenden Erfindung wird vorstehendes Protein mit "SRCR Domäne-enthaltendes Protein" (SRCR-Protein) bezeichnet.

Ein weiterer Gegenstand der vorliegenden Erfindung ist eine für ein (SRCR-Protein) kodierende Nukleinsäure. Dies kann eine RNA oder eine DNA sein. Letztere kann z.B. eine genomische DNA oder eine cDNA sein. Bevorzugt ist eine DNA, die folgendes umfaßt:
(a) die DNA von Fig. 1 oder eine hiervon durch ein oder mehrere Basenpaare unterschiedliche DNA,
(b) eine mit der DNA von (a) hybridisierende DNA, oder
(c) eine mit der DNA von (a) oder (b) über den degenerierten genetischen Code verwandte DNA.

Der Ausdruck "hybridisierende DNA" weist auf eine DNA hin, die unter üblichen Bedingungen, insbesondere bei 20°C unter dem Schmelzpunkt der DNA, mit einer DNA von (a) hybridisiert.

Die DNA von Fig. 1 wurde bei der DSMZ (Deutsche Sammlung von Mikroorganismen und Zellkulturen) als HFL2 unter DSM 11281 am 8. November 1996 hinterlegt.

Nachstehend wird eine erfindungsgemäße Nukleinsäure in Form einer DNA, insbesondere cDNA beschrieben. Diese steht beispielhaft für jede unter die vorliegende Erfindung fallende Nukleinsäure, insbesondere DNA.

Zur Herstellung einer erfindungsgemäßen cDNA ist es günstig, von mRNA aus humaner fötaler Lunge auszugehen. Eine solche mRNA ist bekannt, sie ist z.B. bei Clonetech käuflich erhältlich. Aus der mRNA wird "full-length" cDNA generiert, beispielsweise über oligodT-priming in Kombination mit "Cap-snatching". Der Fachmann ist mit den Verfahren und Bedingungen vertraut. An die "full-length" cDNA wird ein cDNA-Adaptor, beispielsweise aus dem Marathon-Kit von Clonetech, "blunt-end" ligiert. Die cDNA wird dann einem PCR-Verfahren unterzogen, in dem Primerpaare verwendet werden, wobei der eine Primer spezifisch für den cDNA-Adaptor ist und der andere Primer spezifisch für DNA-Sequenzen aus bekannten eine SRCR-Domäne enthaltenden Proteinen ist. Ein Beispiel letzteren Primers ist: cubf1 5'-TGCACATCTCTGAAGACCACAG-3' in 5'-Richtung, und 41nr1 5'-GTGGTCTGCAGGCAGCTG-3' in 3'-Richtung.

Der Primer 41nr1 ist in einem stark konservierten Bereich von SRCR-Domänen lokalisiert, der Primer cubf1 in einem stark konservierten Bereich von CUB-Domänen. Entsprechend der Primerkombination wird eine amplifizierte cDNA erhalten, deren Amplifikation in 5'- bzw. 3'-Richtung erfolgt ist. Die amplifizierte cDNA wird mit einer markierten, für eine erfindungsgemäße Nukleinsäure spezifischen DNA-Sonde hybridisiert. Solche DNA-Sonden sind z.B. die nachstehenden DNA-Sonden aime2e4f1 und a60kexf2:

DNA-Sonde aime2e4f1: 5′-AGGCCAGATACTTGGCTGAC-3′

DNA-Sonde a60kexf2:    5'-CTTCAGATTACTGAAGCCCAGG-3'

Eine cDNA, die mit der DNA-Sonde aime2e4f1 hybridisiert und eine Amplifikation in 5'-Richtung erfahren hat, wird mit einer cDNA ligiert, deren Amplifikation in 3'-Richtung erfolgt ist und die mit der DNA-Sonde a60kexf2 hybridisiert. Das Ligationsprodukt stellt eine erfindungsgemäße cDNA dar.

Eine erfindunggemäße cDNA kann in einem Vektor bzw. Expressionsvektor vorliegen. Beispiele solcher sind dem Fachmann bekannt. lm Falle eines Expressionsvektors für E. coli sind dies z.B. pGEMEX, pUC-Derivate, pGEX-2T, pET3b und pQE-8, wobei letzterer bevorzugt ist. Für die Expression in Hefe sind z.B. pY100 und Ycpad1 zu nennen, während für die Expression in tierischen Zellen z.B. pKCR, pEFBOS, cDM8 und pCEV4, anzugeben sind. Für die Expression in Insektenzeilen eignet sich besonders der Bacculovirus-Expressionsvektor pAcSGHisNT-A.

Der Fachmann kennt geeignete Zellen, um eine, erfindungsgemäße, in einem Expressionsvektor vorliegende cDNA zu exprimieren. Beispiele solcher Zellen umfassen die E.coli-Stämme HB101, DH1, x1776, JM101, JM 109, BL21 und SG 13009, wobei letzterer bevorzugt ist, den Hefe-Stamm Saccharomyces cerevisiae und die tierischen Zellen L, 3T3, FM3A, CHO, COS, Vero und HeLa sowie die lnsektenzellen sf9.

Der Fachmann weiß, in welcher Weise eine erfindungsgemäße cDNA in einen Expressionsvektor inseriert werden muß. Ihm ist auch bekannt, daß diese DNA in Verbindung mit einer für ein anderes Protein bzw. Peptid kodierenden DNA inseriert werden kann, so daß die erfindungsgemäße cDNA in Form eines Fusionsproteins exprimiert werden kann.

Des weiteren kennt der Fachmann Bedingungen, transformierte bzw. transfizierte Zellen zu kultivieren. Auch sind ihm Verfahren bekannt, das durch die erfindungsgemäße cDNA exprimierte Protein zu isolieren und zu reinigen. Ein solches Protein, das auch ein Fusionsprotein sein kann, ist somit ebenfalls Gegenstand der vorliegenden Erfindung.

Ein weiterer Gegenstand der vorliegenden Erfindung ist ein gegen ein vorstehendes Protein bzw. Fusionsprotein gerichteter Antikörper. Ein solcher Antikörper kann durch übliche Verfahren hergestellt werden. Er kann polyklonal bzw. monoklonal sein. Zu seiner Herstellung ist es günstig, Tiere, insbesondere Kaninchen oder Hühner für einen polyklonalen und Mäuse für einen monoklonalen Antikörper, mit einem vorstehenden (Fusions)protein oder Fragmenten davon zu immunisieren. Weitere "Booster" der Tiere können mit dem gleichen (Fusions)protein oder Fragmenten davon erfolgen. Der polyklonale Antikörper kann dann aus dem Serum bzw. Eigelb der Tiere erhalten werden. Für den monoklonalen Antikörper werden Milzzellen der Tiere mit Myelomzellen fusioniert.

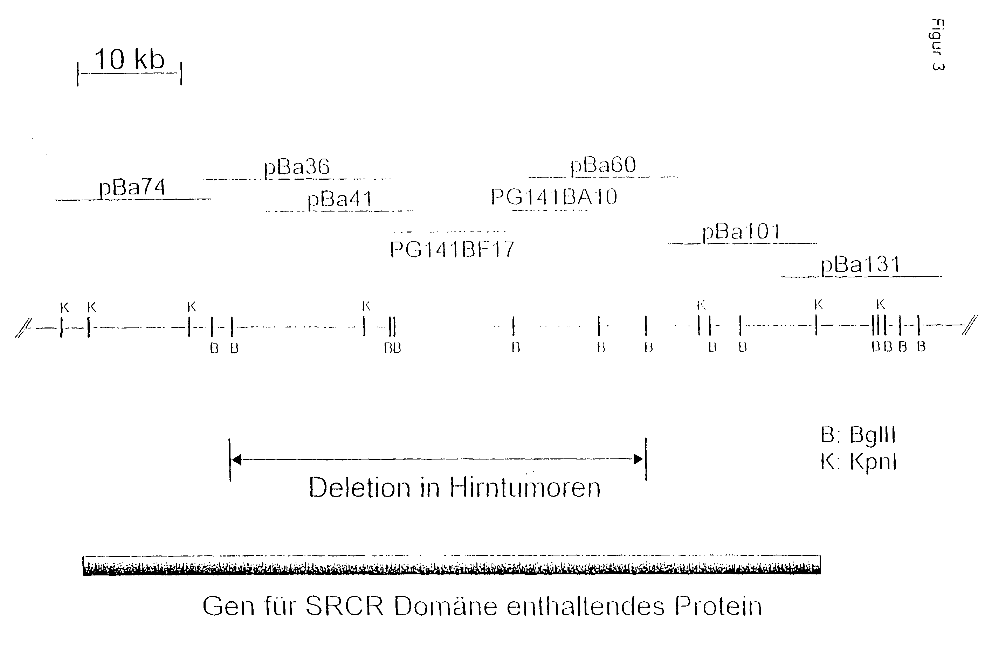

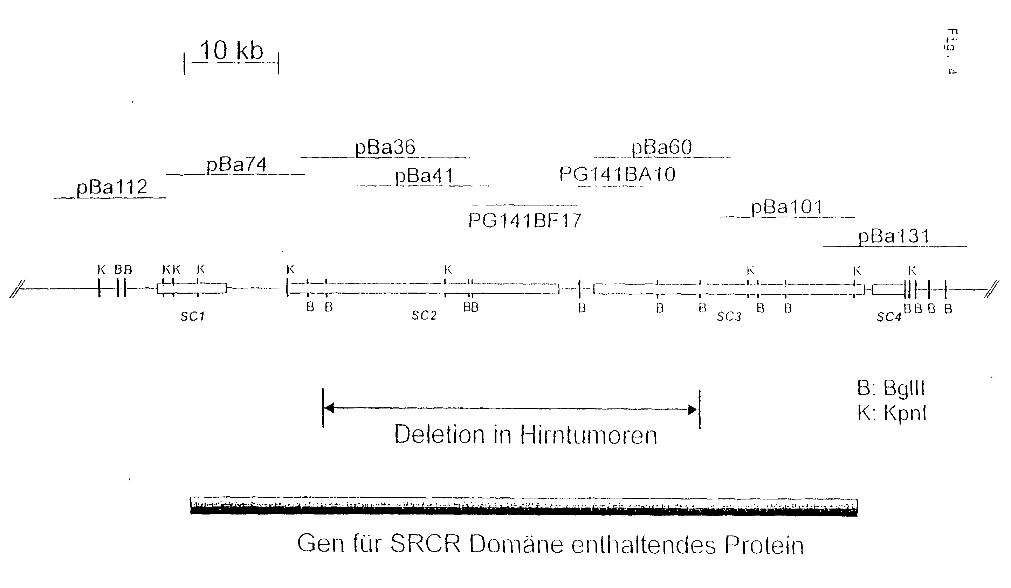

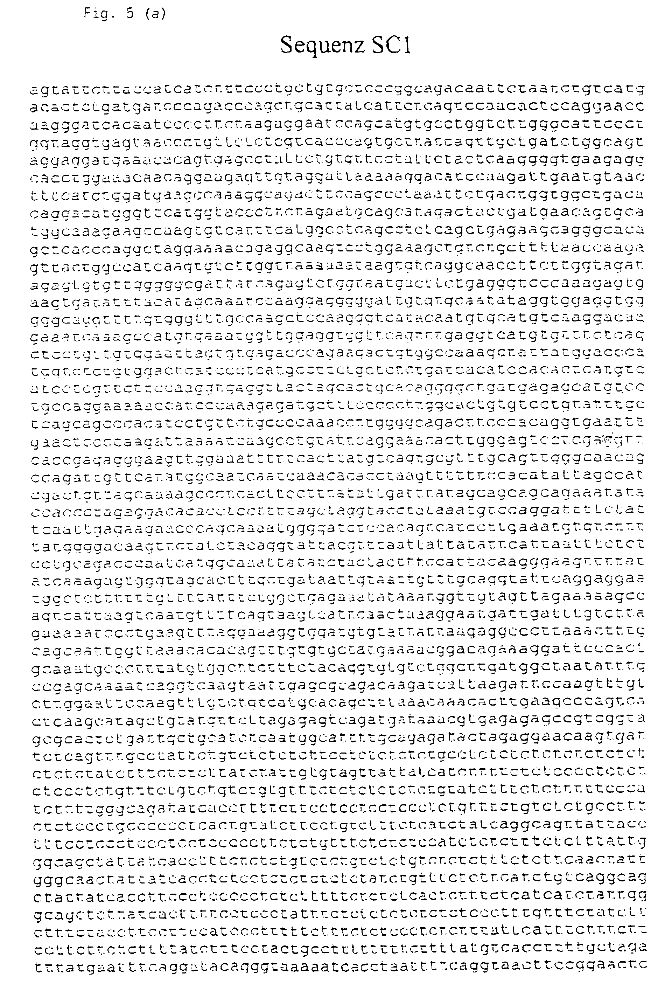

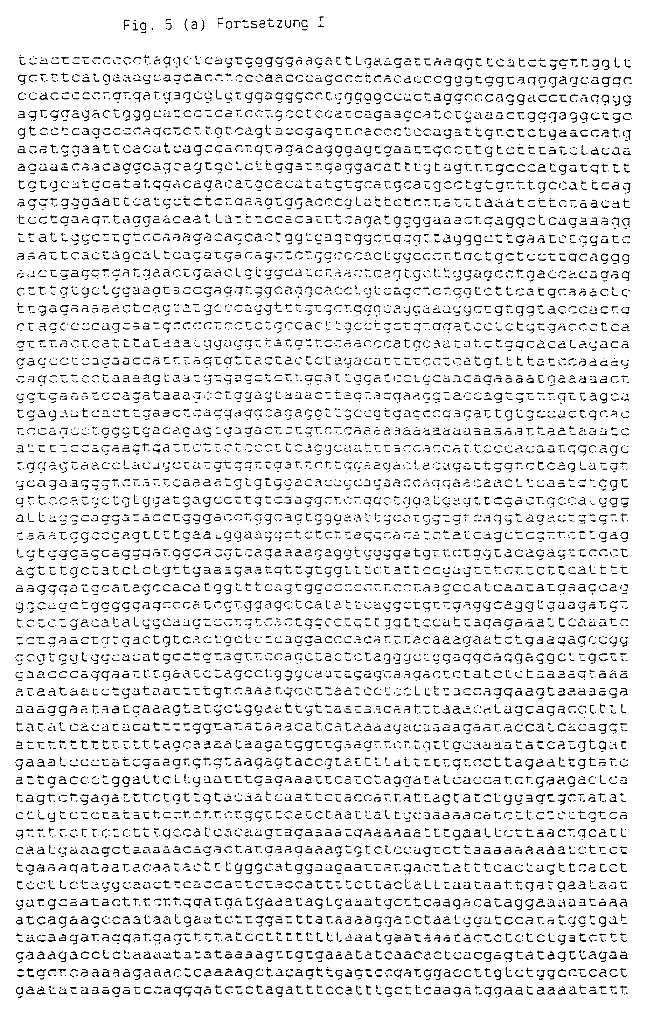

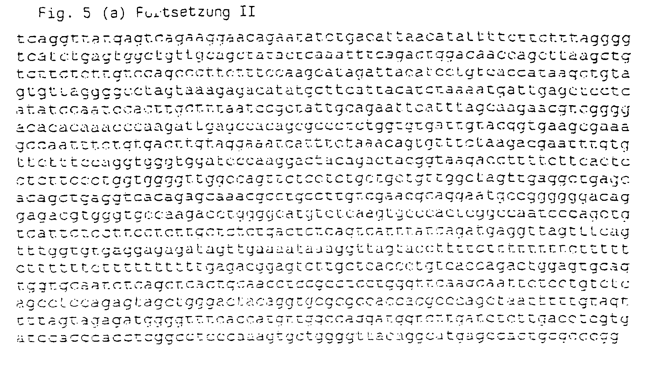

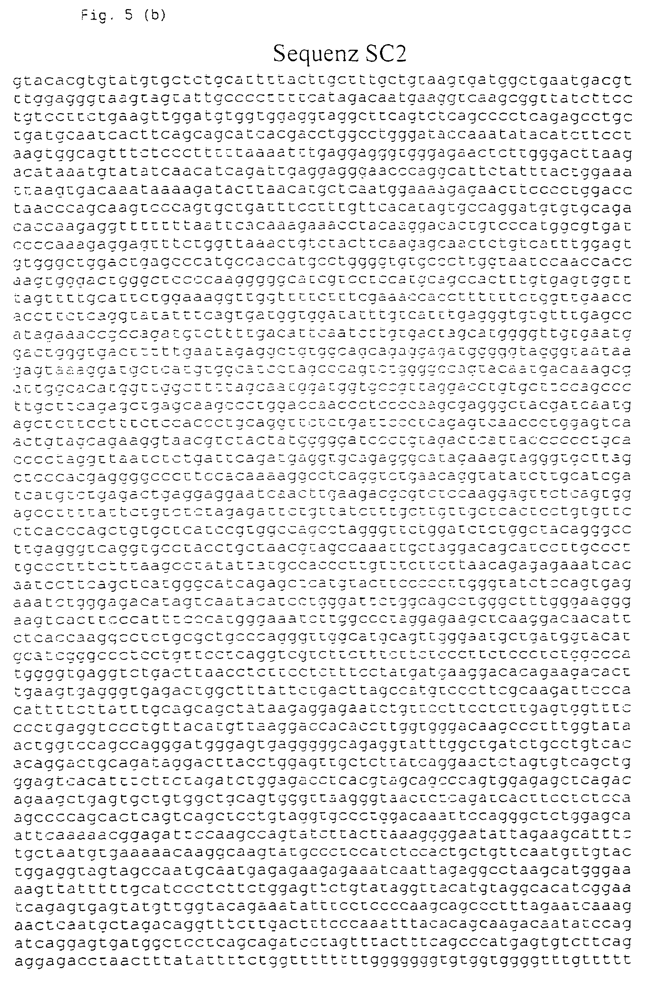

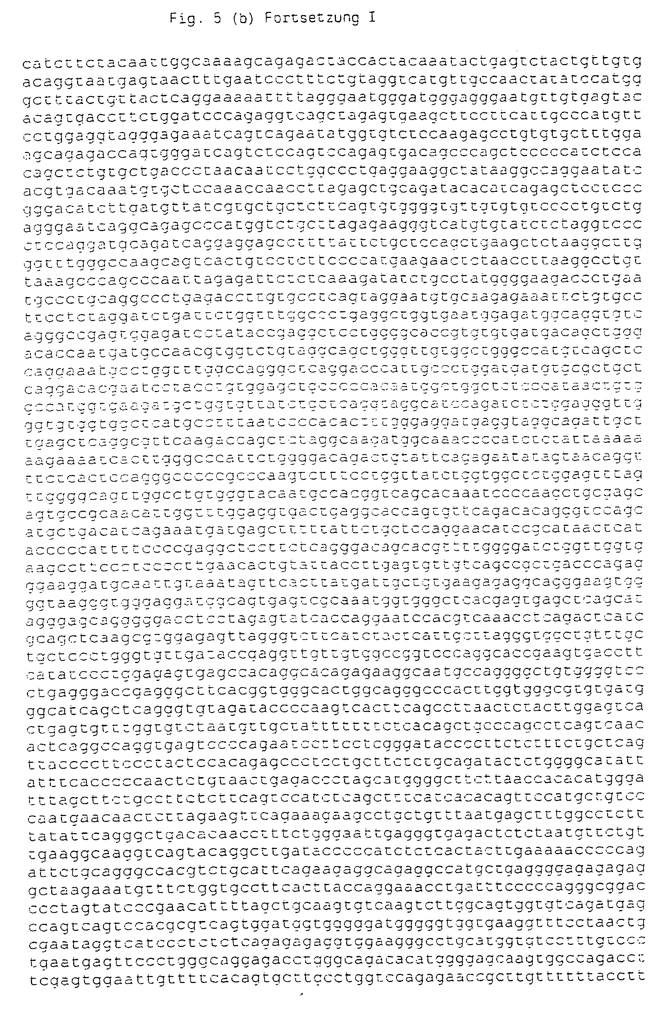

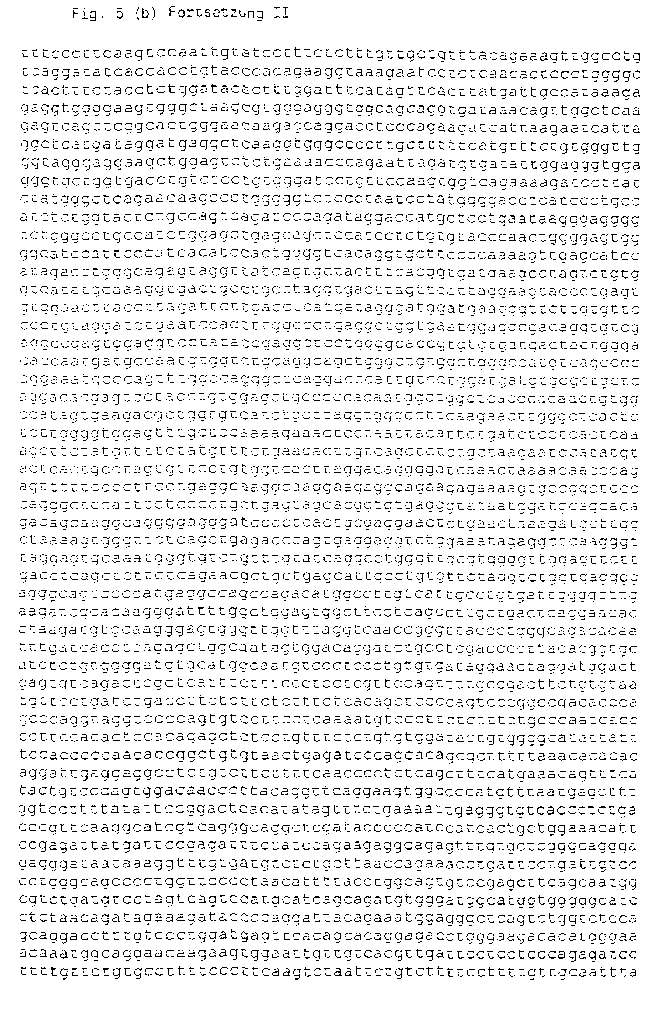

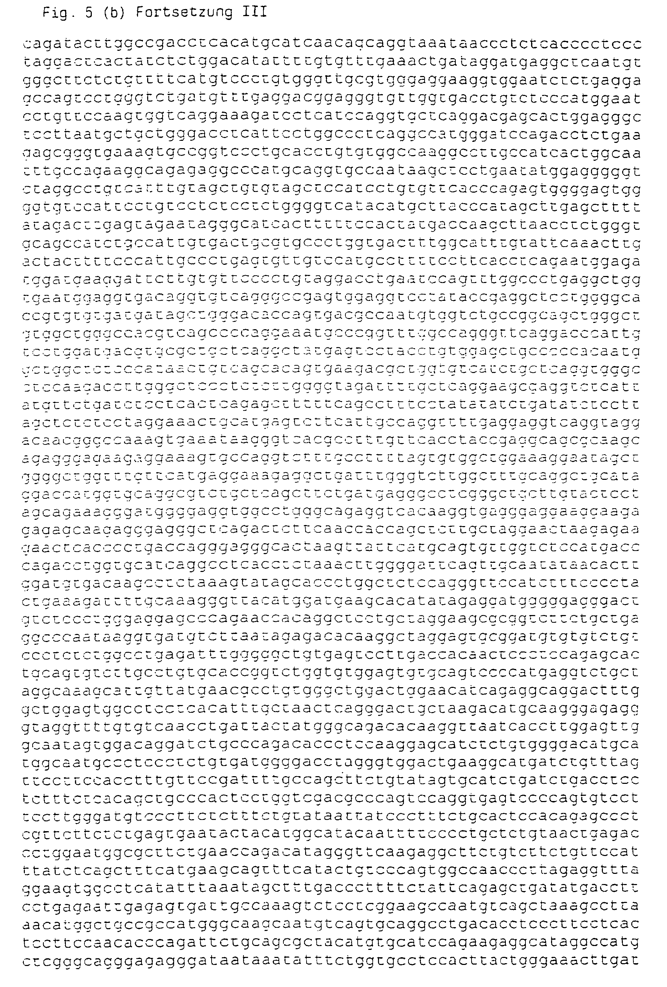

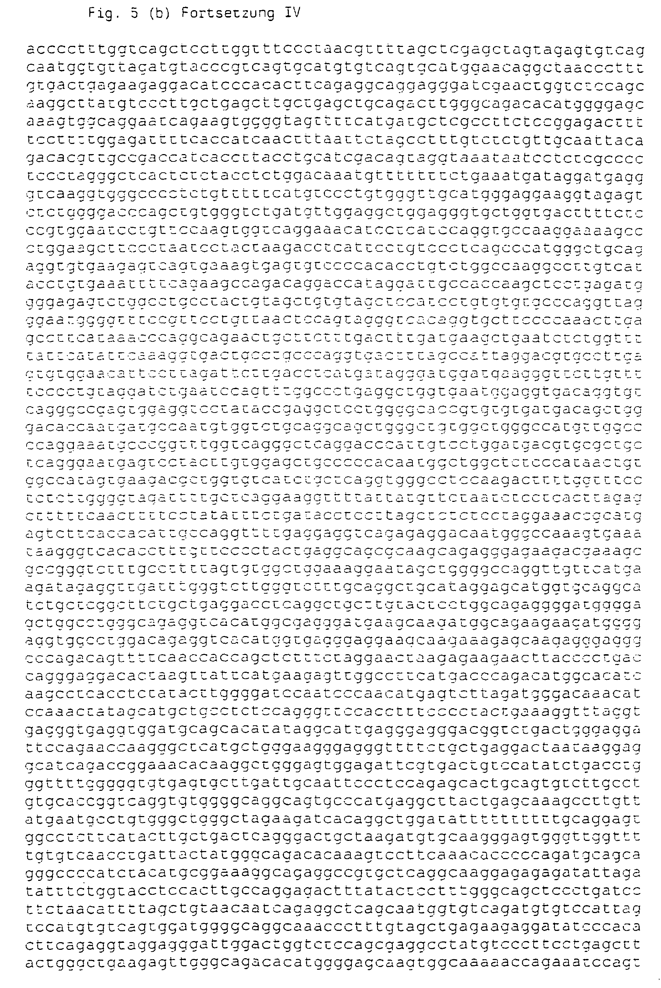

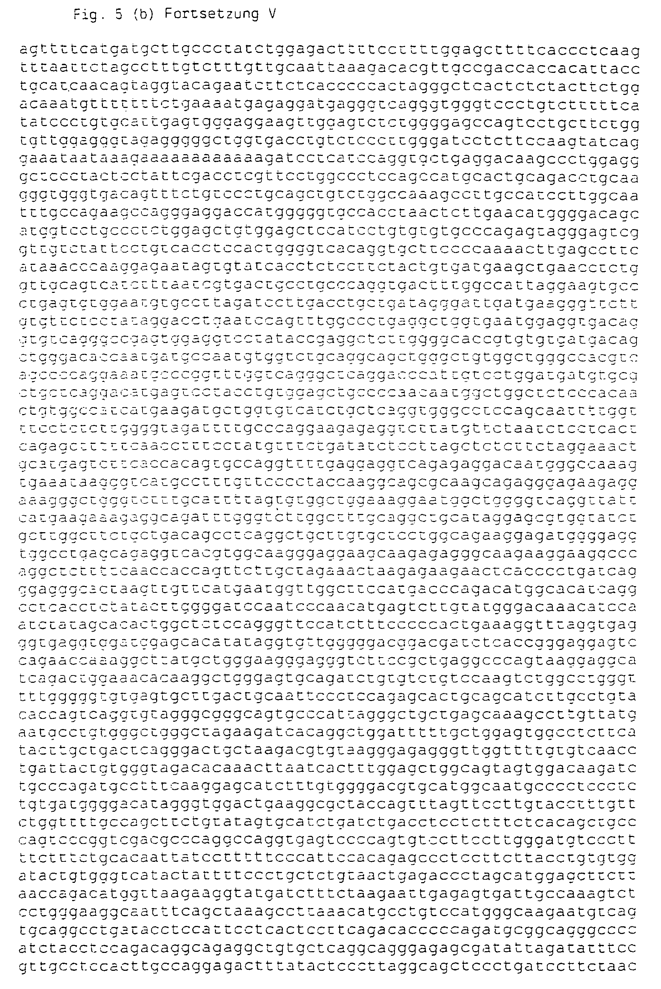

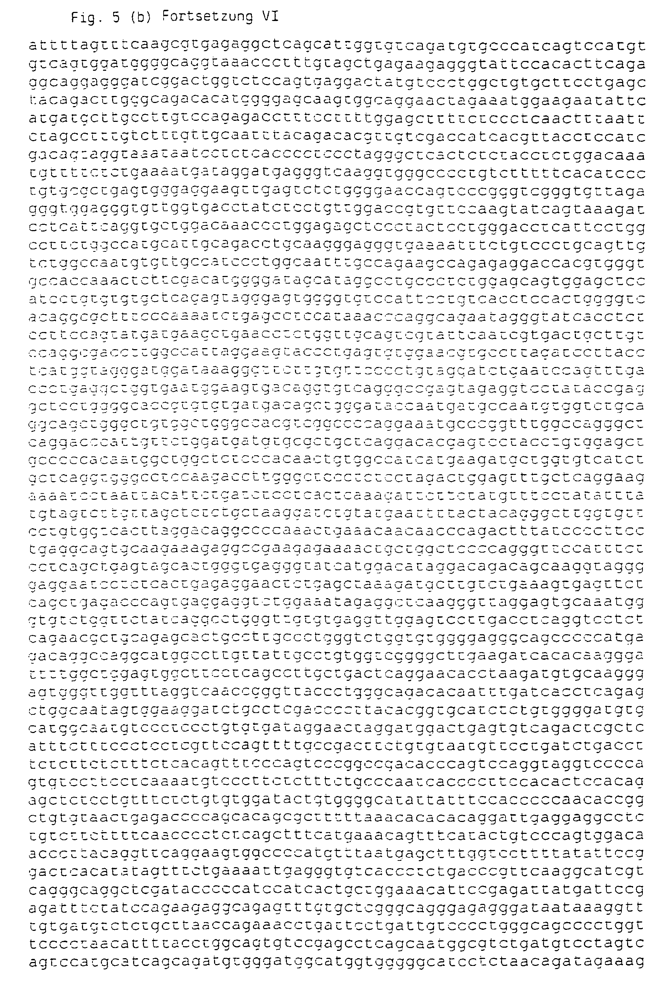

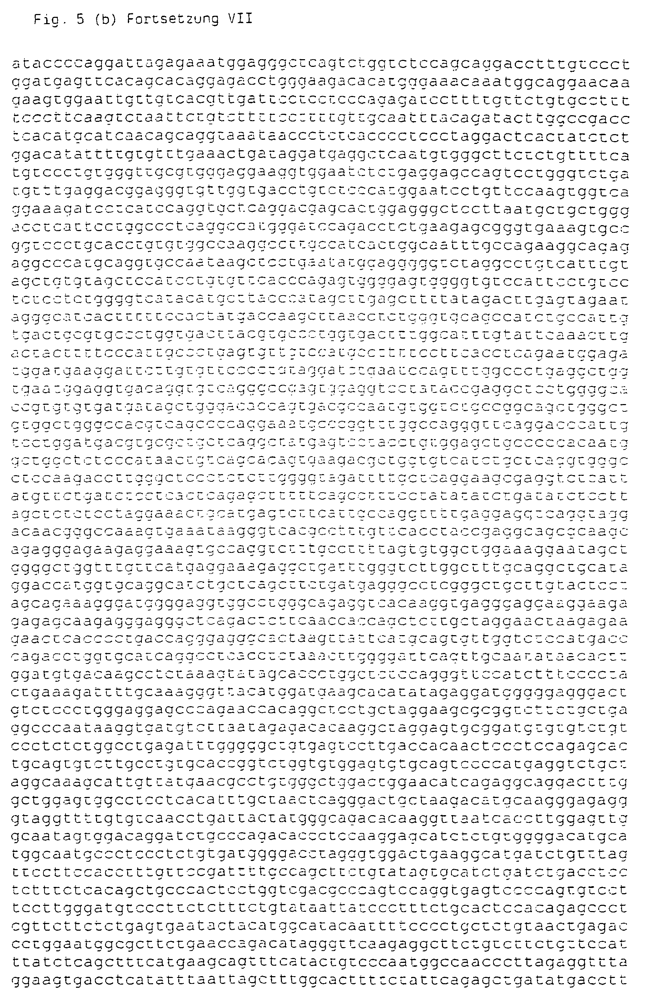

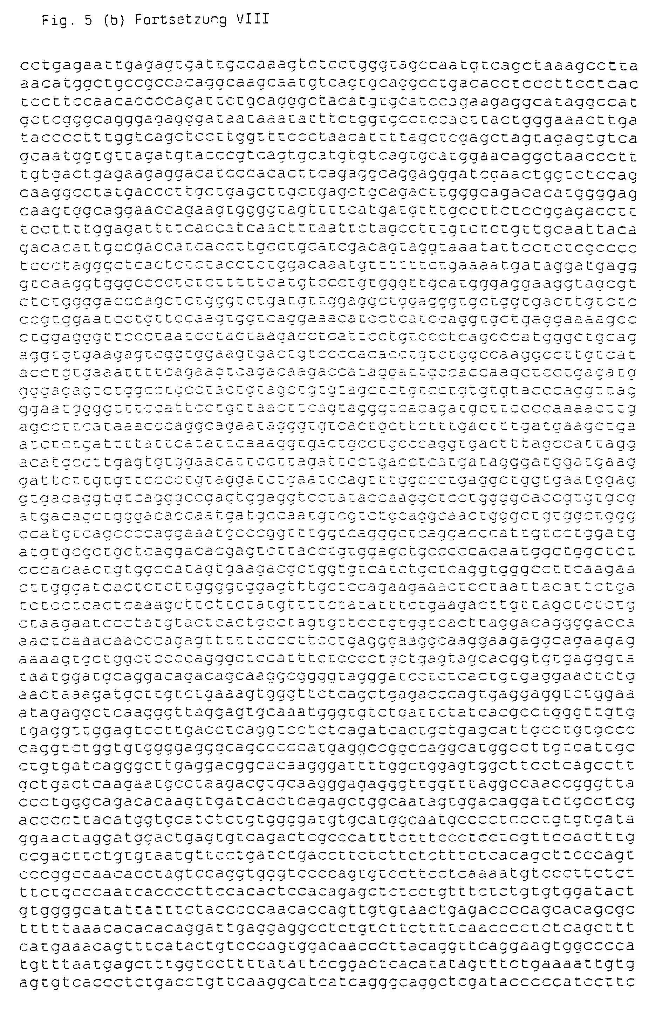

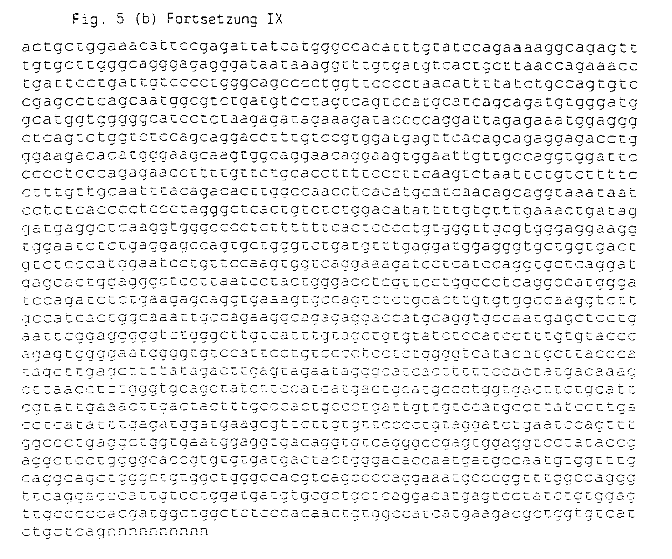

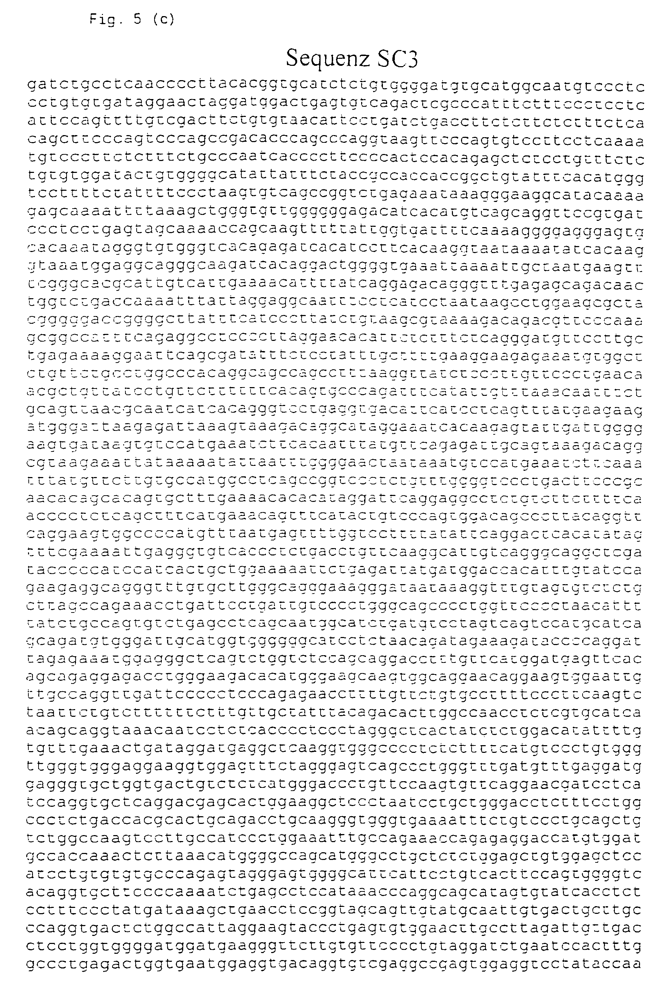

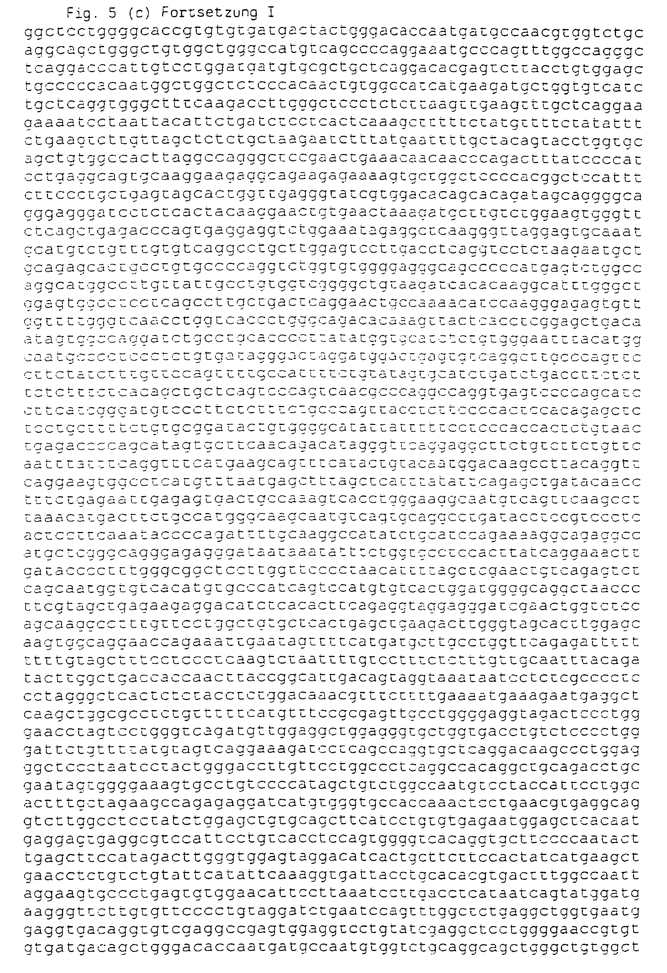

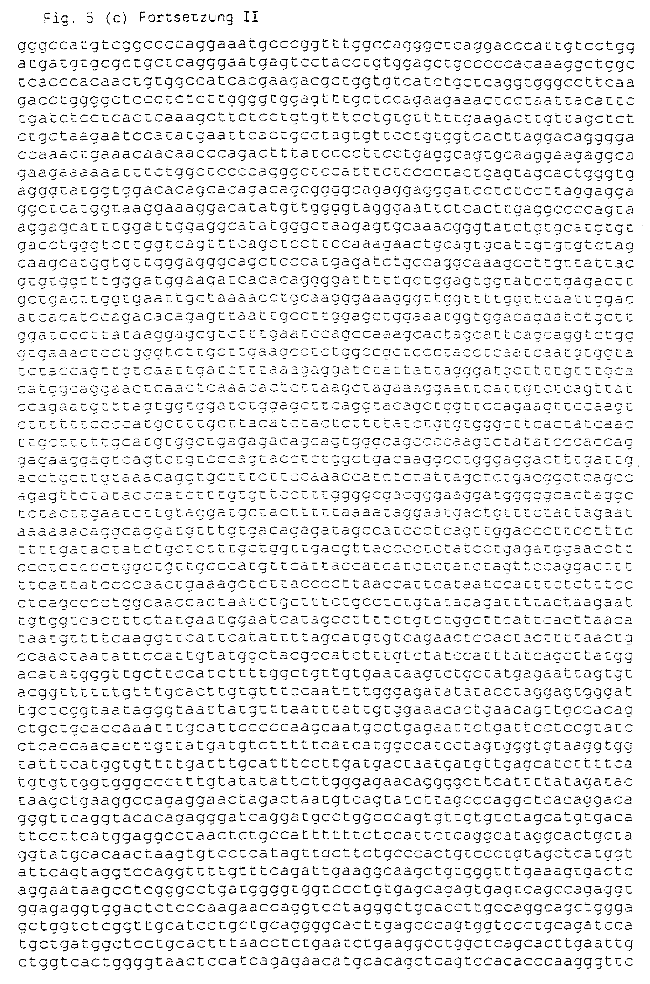

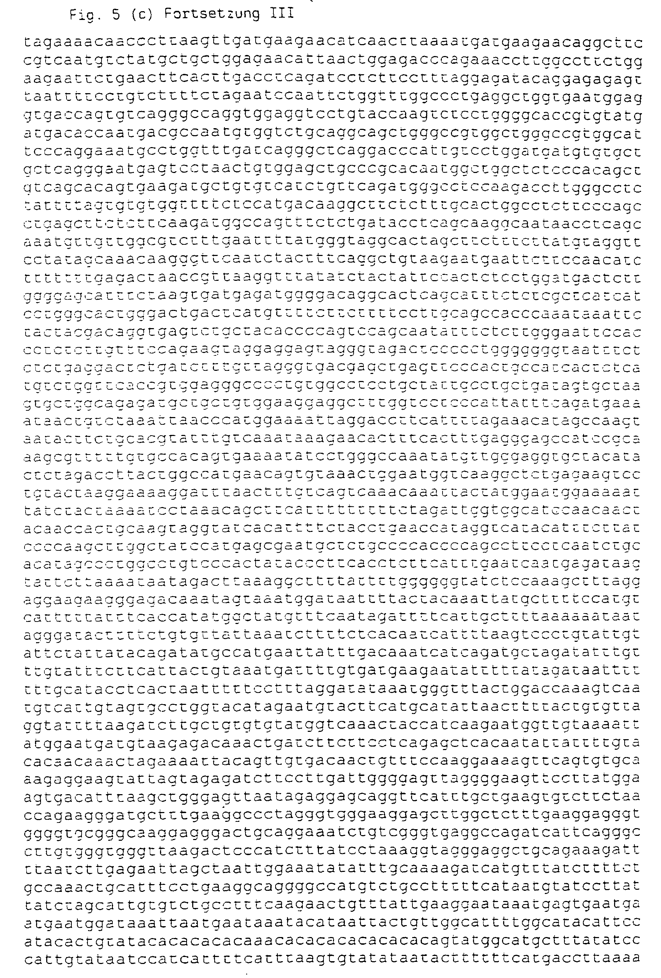

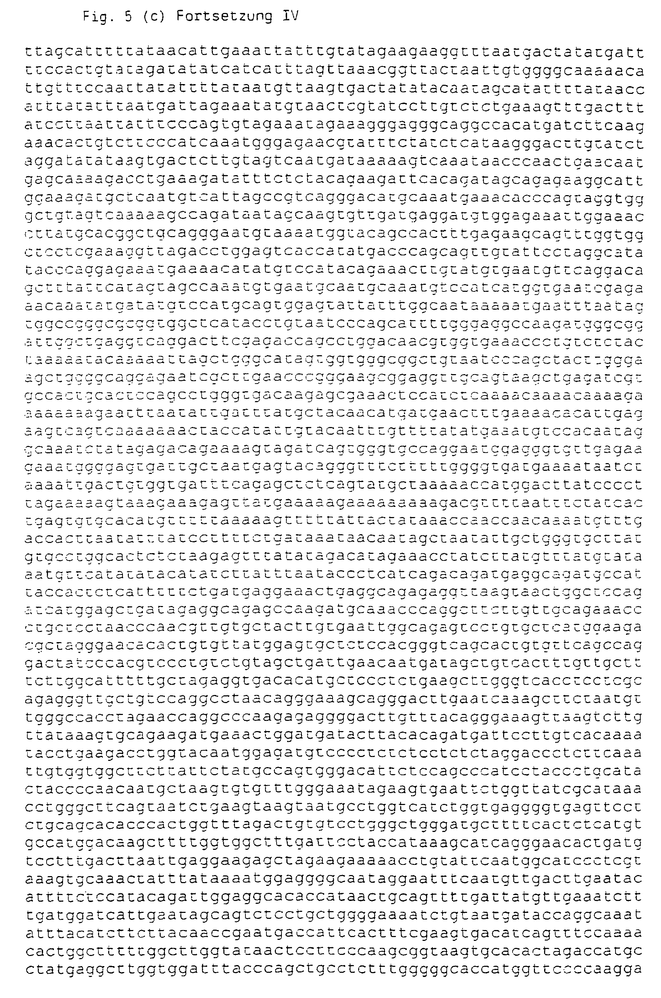

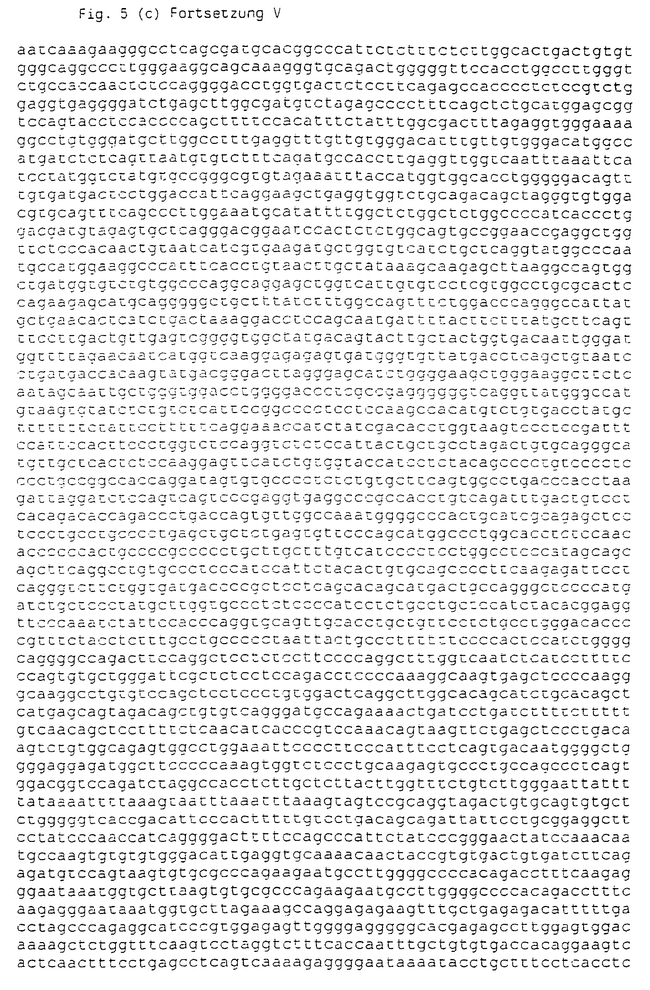

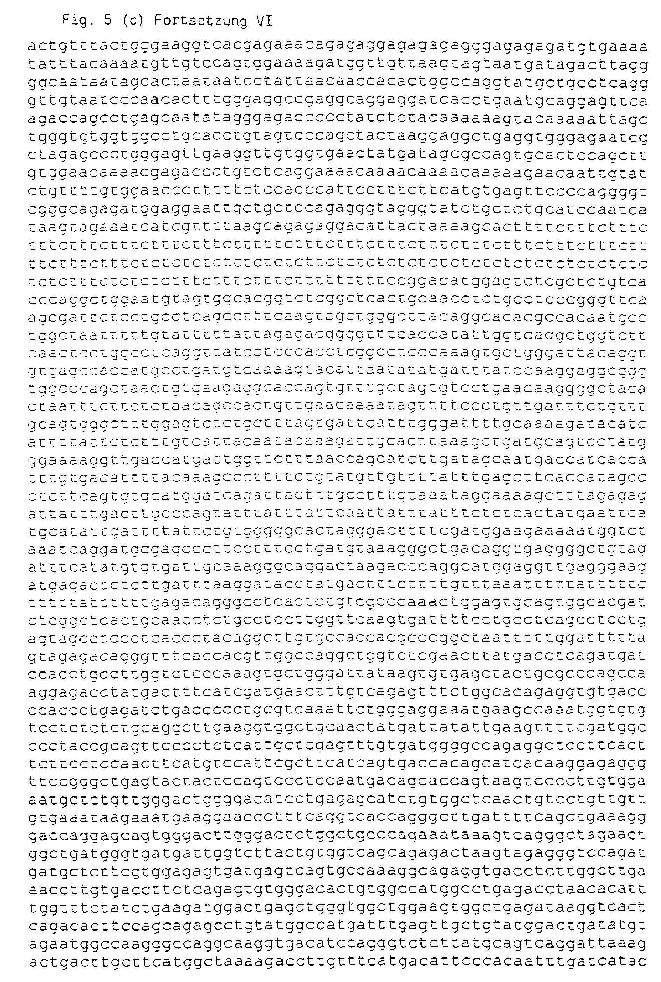

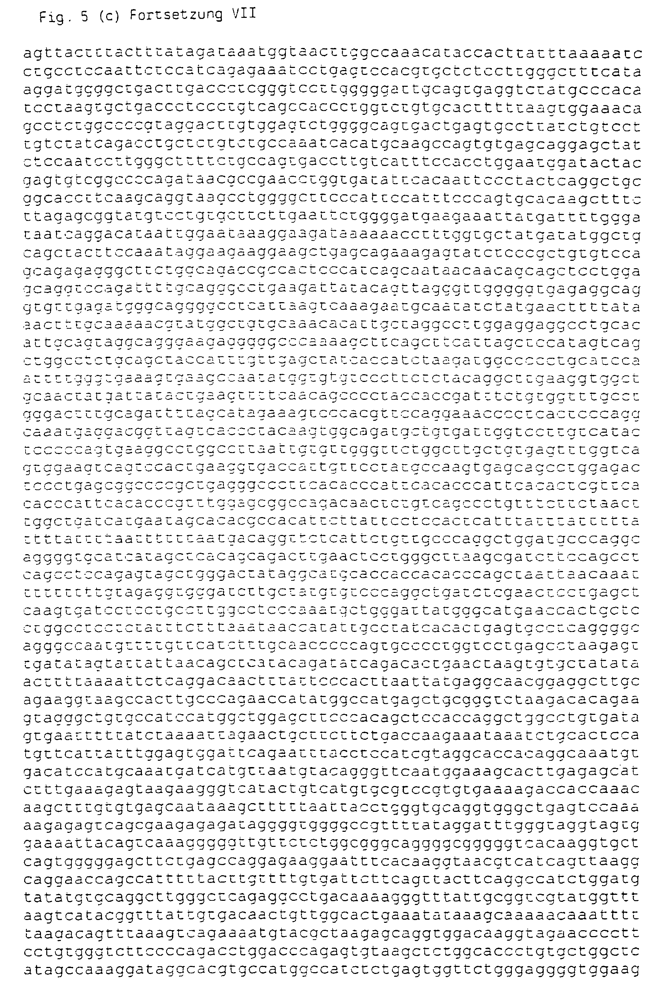

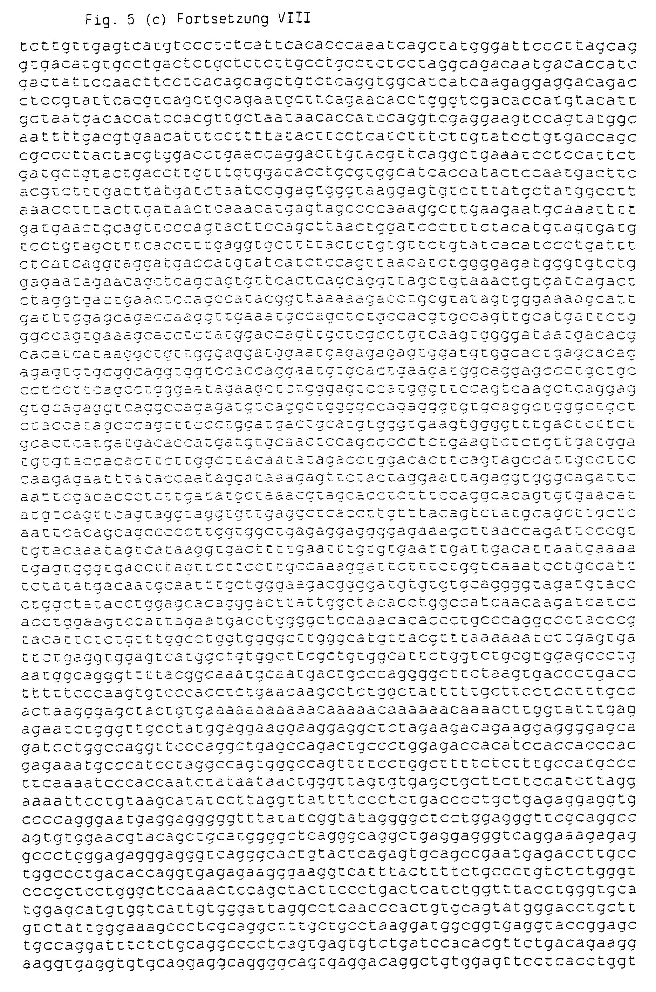

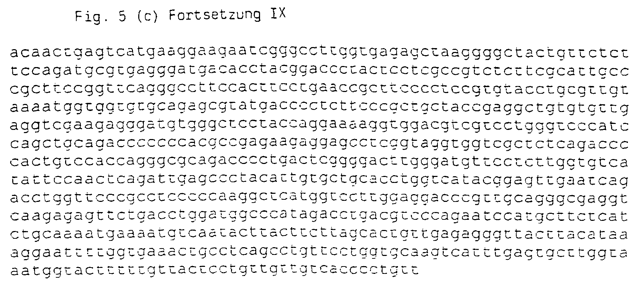

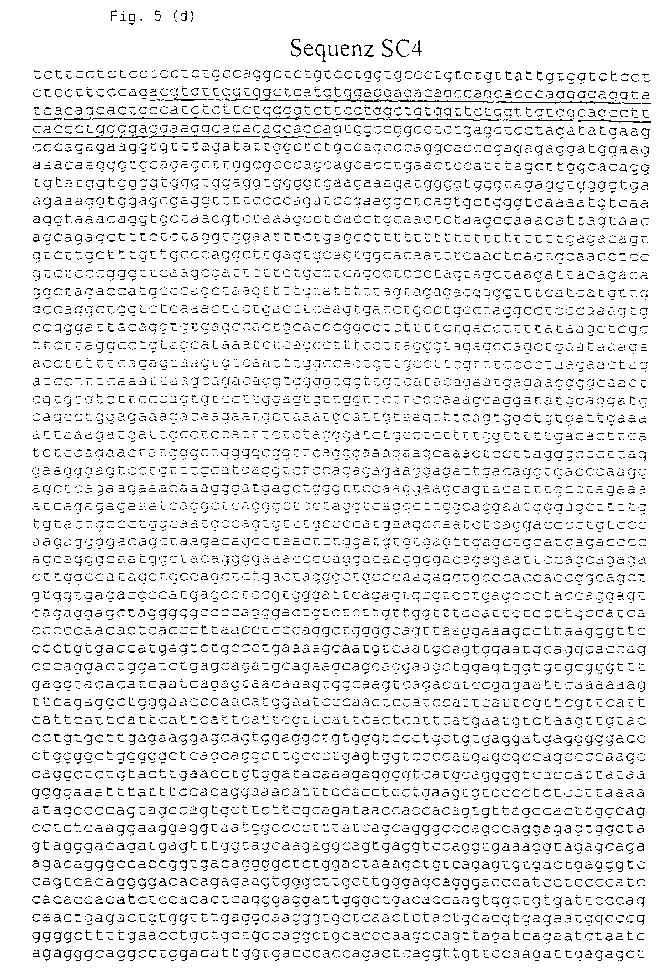

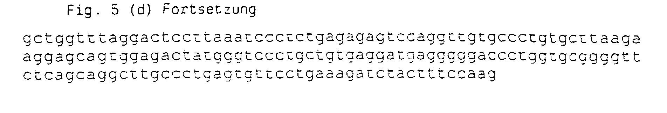

Die vorliegende Erfindung ermöglicht es, elementare Prozesse der Zelle zu untersuchen. Mit einer erfindungsgemäßen Nukleinsäure, insbesondere einer DNA, und hiervon abgeleiteten Primern, kann in Säugetieren, insbesondere dem Menschen, festgestellt werden, ob sie ein Gen enthalten und/oder exprimieren, das für ein (SRCR-Protein) kodiert. Ferner kann festgestellt werden, in welcher Form das (SRCR-Protein) vorliegt und welche Bedeutungen die einzelnen Formen für die Zelle bzw. Prozessen dieser haben. Beispielsweise hat der Anmelder gefunden, daß in Medulloblastomen und Glioblastomen sowie bei Brustkrebs das (SRCR-Protein) eine Deletion aufweist. Diese Deletion kann auch auf genomischer Ebene nachgewiesen werden. Es werden die genomischen DNA-Klone pBa 112, pBa74, pBa36, pBa41, PG141BF17, PG141BA10, pBa60, pBa101 und pBa131 bereitgestellt, die in der aufgeführten Reihenfolge einen DNA-Bereich von 5' - > 3' angeben (vgl. Fig. 3, 4 und 5), der in nomalen Zellen vorhanden ist, jedoch in Tumorzellen, insbesondere in Zellen eines Modulloblastoms, eines Glioblastoms oder von Brustkrebs, auf beiden Allelen Deletionen aufweist. Die genannten DNA-Klone sind ebenfalls Gegenstand der vorliegenden Erfindung. Sie wurden auch bei der DSMZ hinterlegt: pBa74 unter DSM 11280, pBa36 unter DSM 11277, pBa41 unter DSM 11278 und pBa60 unter DSM 11279, jeweils am 8. November 1996; sowie PG141BF17 unter DSM 11649, PG141BA10 unter DSM 11648, pBa101 unter DSM 11646 und pBa131 unter DSM 11647, jeweils am 04. Juli 1997, Die genomischen Klone stellen zusätzlich zu der kodierenden Sequenz Intron-Sequenzen zur Verfügung. Diese sind zur Mutationsanalyse in Tumoren, z.B. als Hybridisierungssonden oder mit daraus abgeleiteten Primern, geeignet. Die genomischen Klone stellen somit diagnostische Mittel zur Verfügung. Zur Durchführung vorstehender Untersuchungen können übliche Verfahren, wie Reverse Transkription, PCR-Reaktion, Hybridisierung und Sequenzierung, herangezogen werden. Erfindungsgemäß wird auch ein Kit bereitgestellt, der eine vorstehende Nukleinsäure, insbesondere DNA, und/oder hiervon abgeleitete Primer sowie Träger und übliche Hilfsstoffe enthält. Desweiteren können mit einem erfindungsgemäßen (SRCR-Protein) diagnostische Maßnahmen ergriffen werden. Insbesondere kann festgestellt werden, ob Autoantikörper gegen ein solches Protein existieren.

Ferner eignet sich die vorliegende Erfindung, in elementare Prozesse der Zelle einzugreifen. Ein (SRCR-Protein) kann in Säugetieren, insbesondere den Menschen, eingebracht werden. Hierzu kann es günstig sein, das (SRCR-Protein) an ein vom jeweiligen Körper nicht als fremd angesehenes Protein, z.B. Transferrin oder BSA, zu koppeln. Auch kann eine erfindungsgemäße Nukleinsäure, insbesondere eine DNA, in Säugetiere, insbesondere den Menschen, eingebracht und dort exprimiert werden. Hierzu kann es günstig sein, die Expression der erfindungsgemäßen Nukleinsäure unter die Kontrolle eines Gewebe-spezifischen Promotors zu stellen. Mit einem erfindungsgemäßen Antikörper kann die Expression eines (SRCR-Protein) kontrolliert und reguliert werden.

Die vorliegende Erfindung stellt somit einen großen Beitrag zur diagnostischen und therapeutischen Erfassung von elementaren Prozessen der Zelle dar. Insbesondere können Tumorerkrankungen, ganz besonders des zentralen Nervensystems, z.B. Medulloblastome oder Glioblastome, sowie der Brust auf genetischer Ebene diagnostiziert und Maßnahmen dagegen ergriffen werden. Die diagnostische Erfassung elementarer Prozesse der Zelle kann dabei nicht nur post- sondern bereits auch pränatal erfolgen.

**Kurze Beschreibung der Zeichnungen:**
- Fig. 1: zeigt die Basensequenz und die davon abgeleitete Aminosäuresequenz, die von einem erfindungsgemäßen (SRCR-Protein) umfaßt wird,

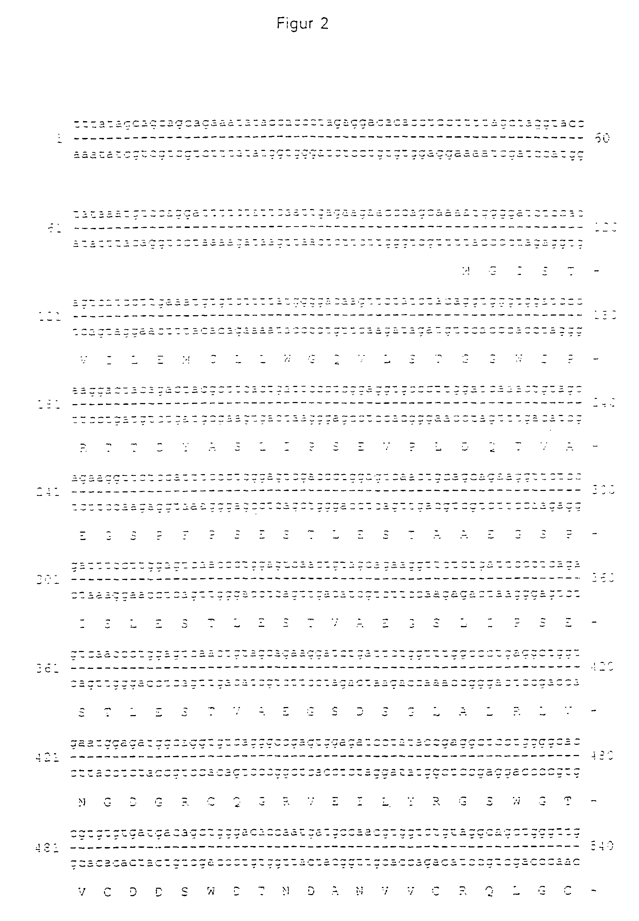

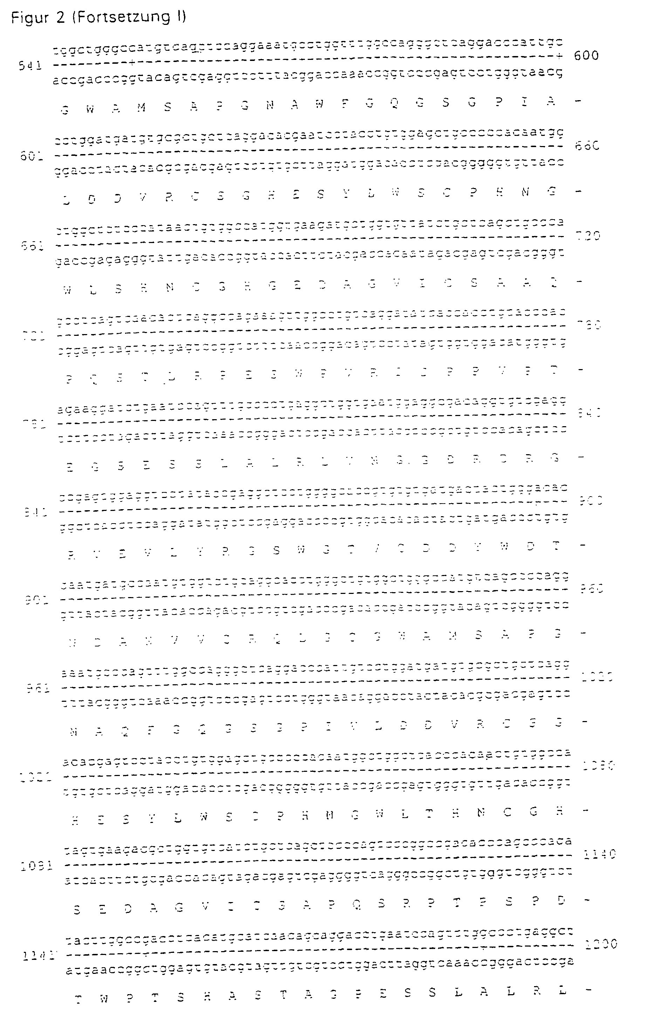

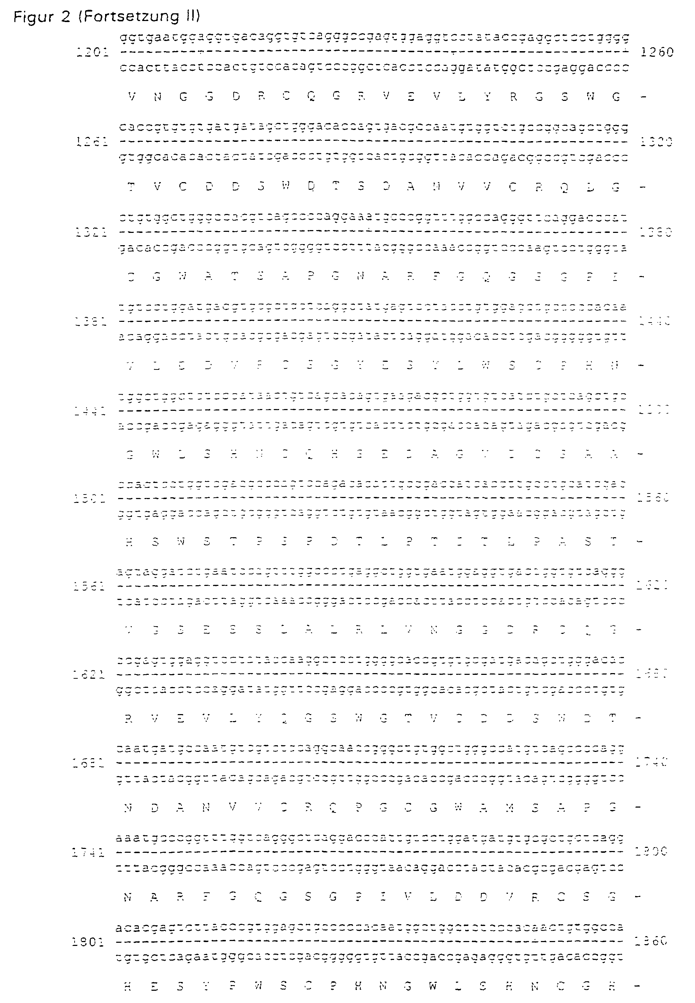

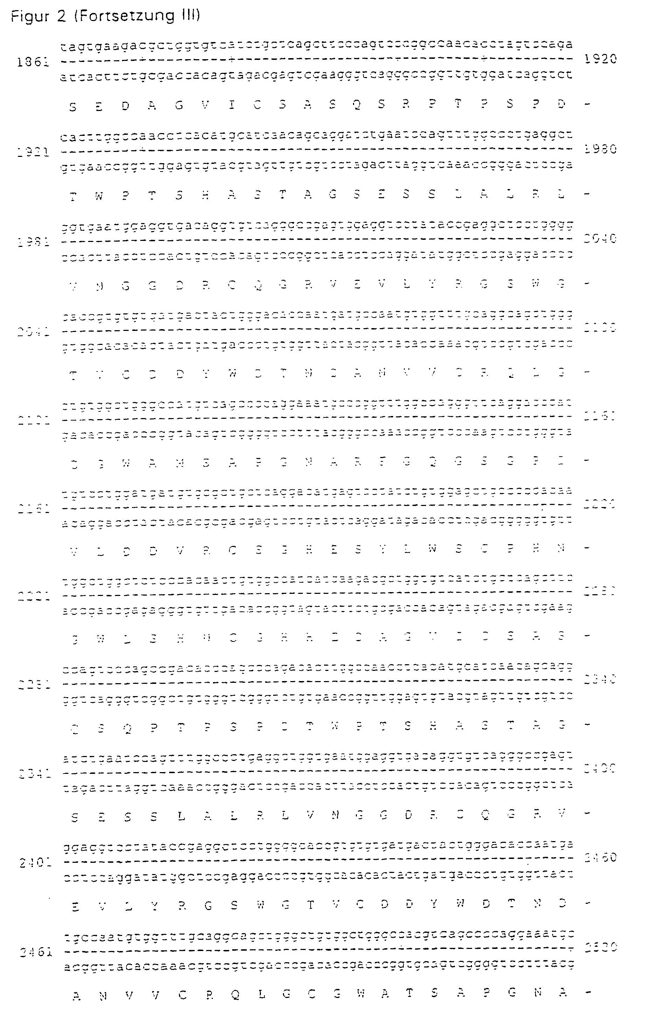

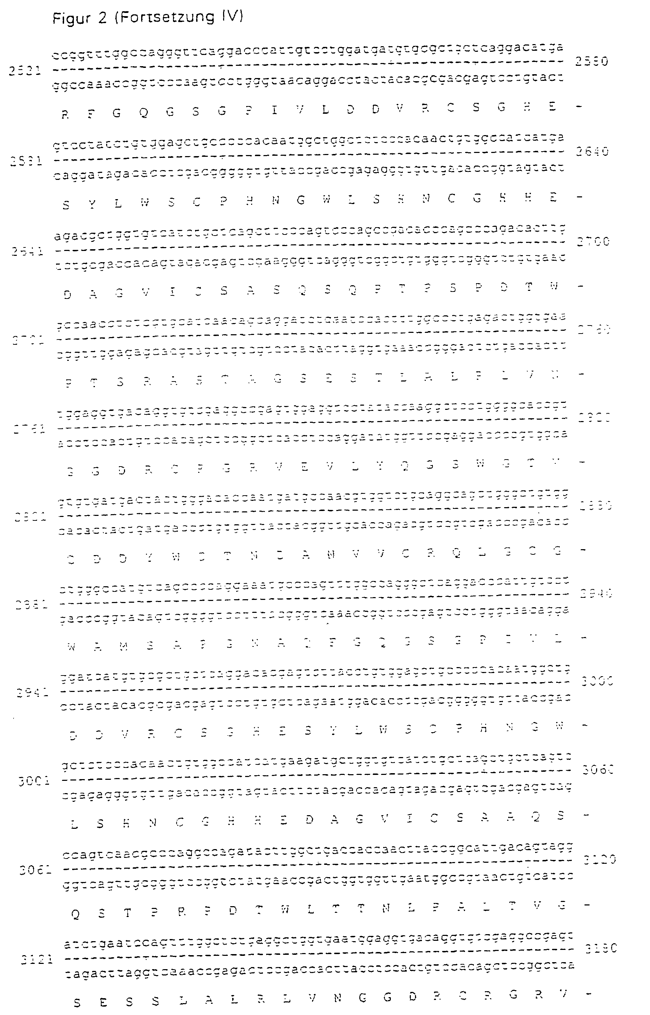

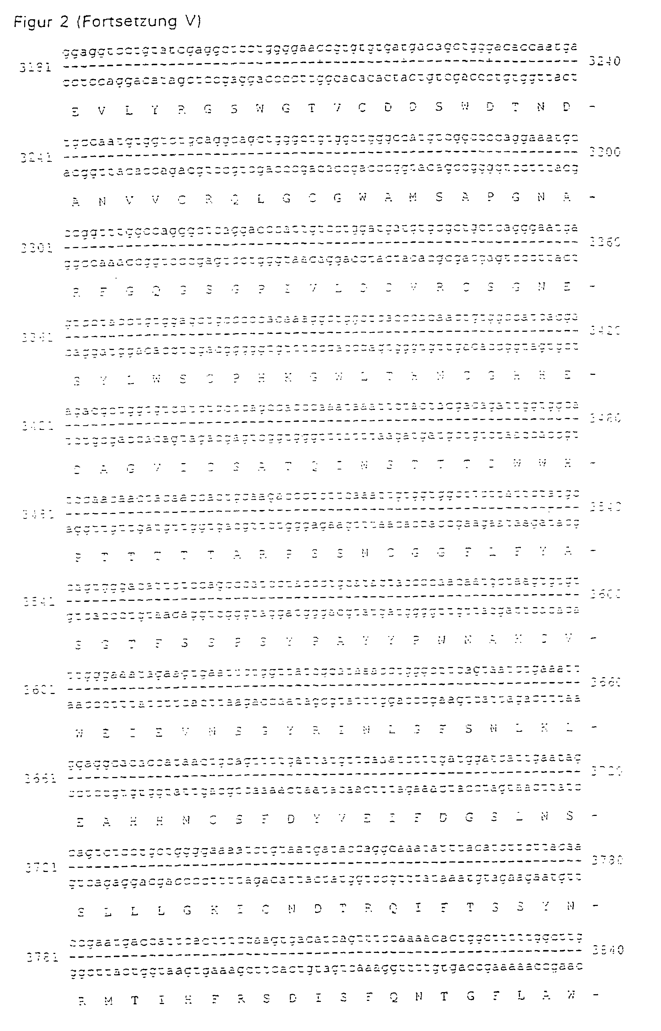

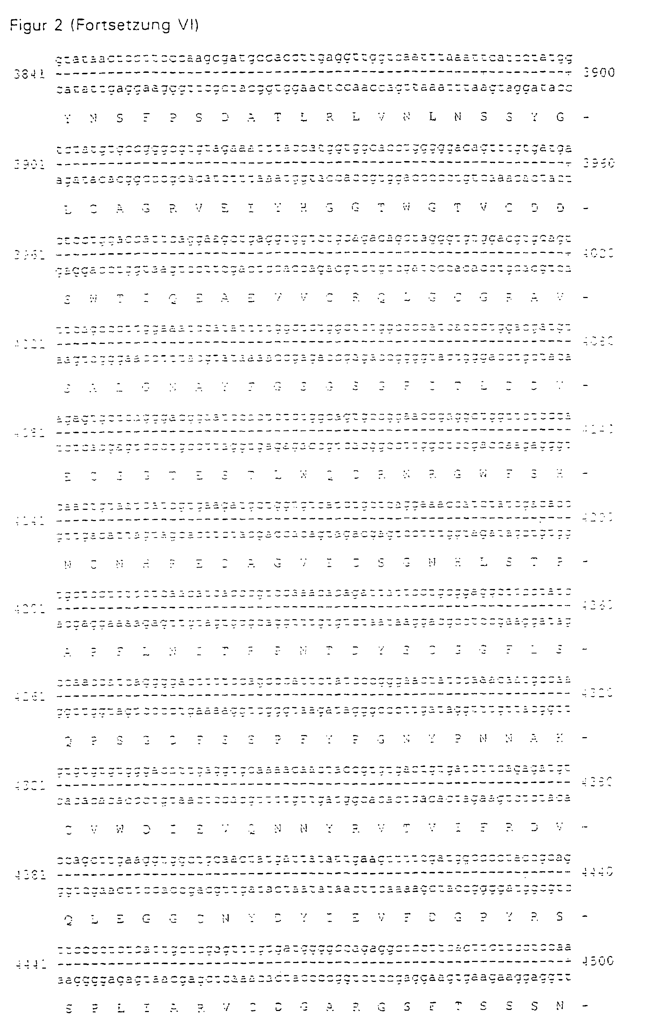

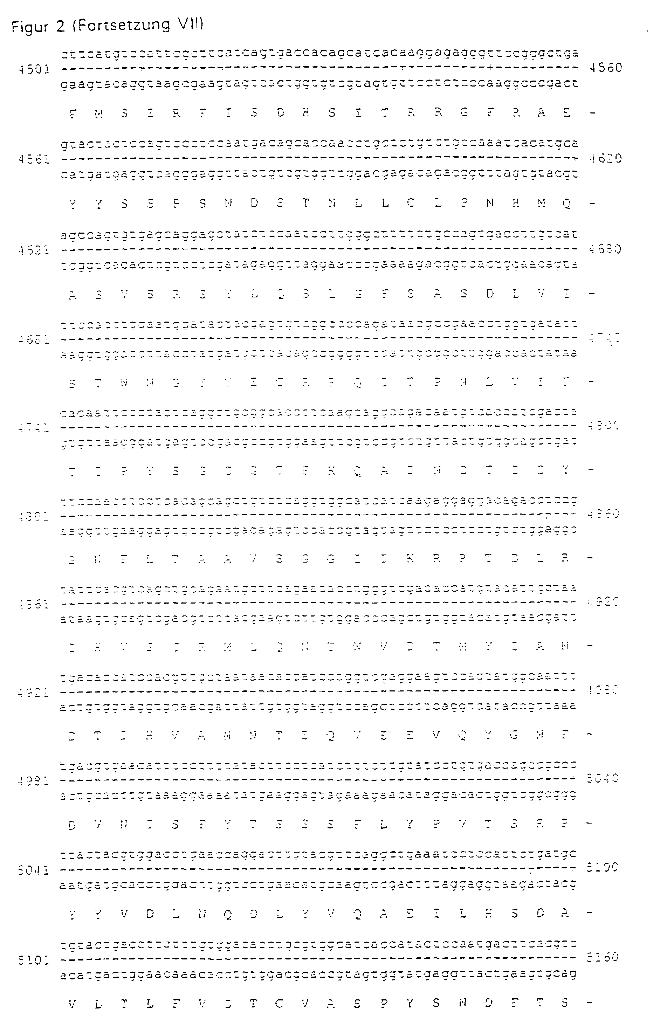

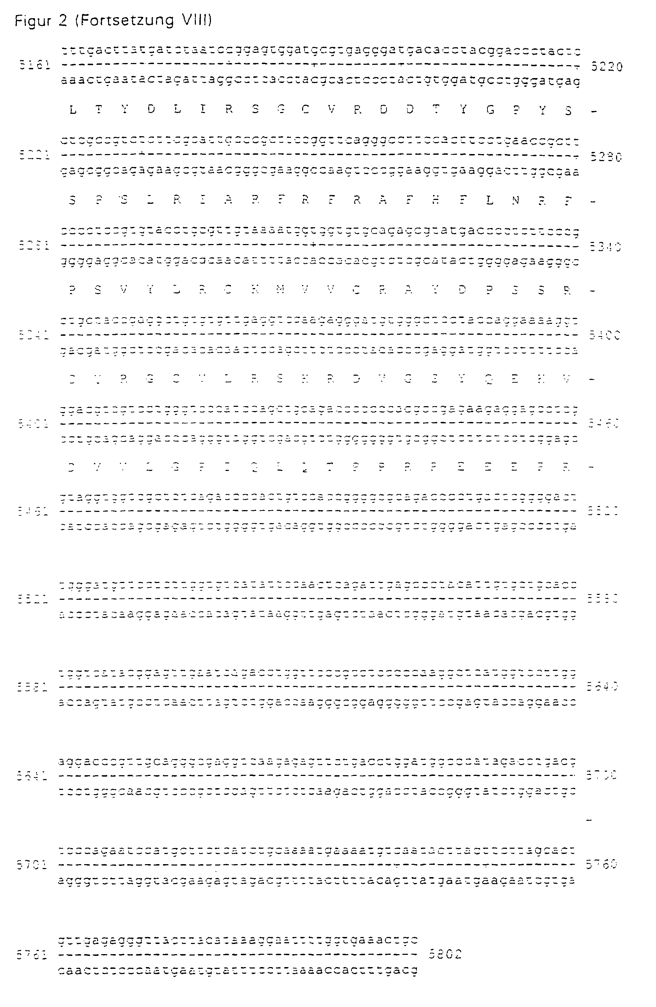

- Fig. 2: zeigt die Basensequenz und die davon abgeleitete Aminosäuresequenz, die von einem erfindungsgemäßen (SRCR-Protein) umfaßt wird, wobei vorstehende Fig. 1 einen Ausschnitt von Fig. 2 darstellt (Nucleotide 2958 bis 4957),
- Fig. 3: zeigt die Restriktionskarte und die Relation der genomischen Klone pBA74, pBa36, pBa41, PG141BF17, PG141BA10, pBa60, pBa101 und pBa131 zueinander,
- Fig. 4: zeigt die Restriktionskarte und die Relation der genomischen Klone pBa112, pBA74, pBa36, pBa41, PG141BF17, PG141BA10, pBa60, pBa101 und pBa131 zueinander. Ferner sind die durch diese Klone umfaßten Sequenzbereiche SC 1, SC2, SC3 und SC4 angegeben, und
- Fig. 5: zeigt die Sequenz der Sequenzbereiche SC1, SC2, SC3 und SC4 (Fig. 5(a), (b), (c), (d)). Ferner ist in SC4 ein Bereich durch Unterstreichung gekennzeichnet, der für eine Transmembrandomäne kodiert.

Die vorliegende Erfindung wird durch die nachstehenden Beispiele erläutert.

### Beispiel 1: Herstellung und Reinigung eines erfindungsgemäßen (SRCR-Protein)

Zur Herstellung eines erfindungsgemäßen (SRCR-Protein) wurde eine mRNA aus humaner fötaler Lunge (Clonetech) verwendet. Aus der mRNA wurde "full-length" cDNA hergestellt, wobei oligodT-priming in Kombination mit "Cap-snatching" durchgeführt wurde. An die "full-length" cDNA wurde ein cDNA-Adaptor aus dem Marathon-Kit von Clonetech "blunt-end" ligiert. Die cDNA wurde dann einem PCR-Verfahren unterzogen. Als Primerpaar wurden verwendet: cubf1 5'-TGCACATCTCTGAAGACCACAG-3' in 5'-Richtung, bzw. 41nr1 5'-GTGGTCTGCAGGCAGCTG-3' in 3'-Richtung, jeweils in Kombination mit einem für den cDNA-Adaptor spezifischen Primer.

Der PCR-Ansatz bzw. die PCR-Bedingungen waren wie folgt:

| PCR-Ansatz: | |
|---|---|
| Template-DNA | 1µl = 10ng |
| 10x TaKARa long-range PCR-Puffer | 2.5µl |
| MgCl₂ (25mM) | 2.5µl |
| Taq/Pfu-Polymerase-Mix (19:1) | 0,25µl |
| dNTP-Mix (10mM jedes) | 1µl |
| Oligonukleotide (10pmol/µl | 1µl |
| H₂O bidest. | ad 25µl |

| PCR-Bedingungen: | |
|---|---|
| 95°C 3 min | 1 Zyklus |
| | |
| 94°C 15 sec | |
| 60°C 10 sec | |
| 68°C 4 min | 15 Zyklen |
| | |
| 94°C 15 sec | |
| 60°C 10 sec | |
| 68°C 4 min | 10 Zyklen mit einer Verlängerung von je 10 sec des 68°C-Schrittes pro Zyklus. |

Die amplifizierte cDNA wurde einer Hybridisierung mit den vorstehend angegebenen DNA-Sonden aime2e4f1 und a60kexf2 unterzogen. Ihre Sequenzen sind spezifisch zu dem vorstehend angegebenen genomischen DNA-Klon pBa60.
Es wurde cDNA identifiziert, die mit den DNA-Sonden hybridisierte. Diese cDNA hatte eine Amplifikation in 5'- bzw. 3'-Richtung erfahren. Die cDNA wurde über eine Agarose-Gelelektrophorse gereinigt und mit Swal gespalten. Nach weiterer Agarose-Gelektrophorese wurde eine in 5'Richtung amplifizierte cDNA mit einer in 3'-Richtung amplifizierten ligiert. Das Ligationsprodukt wurde sequenziert. Es umfaßte die DNA von Fig. 1, Das Ligationsprodukt wurde mit Notl gespalten und in den mit Notl gespaltenen Expressionsvektor pQE-30 NST (Quiagen) inseriert. Es wurde das Expressionsplasmid pQ/SRCR-Protein erhalten. Ein solches kodiert für ein Fusionsprotein aus 6 Histidin-Resten (N-Terminuspartner) und einem erfindungsgemäßen (SRCR-Protein) (C-Terminuspartner). pQ/SRCR-Protein wurde zur Transformation von E.coli SG 13009(vgl. Gottesman, S. et al., J. Bacteriol. 148, (1981), 265-273) verwendet. Die Bakterien wurden in einem LB-Medium mit 100µg/ml Ampicillin und 25µg/ml Kanamycin kultiviert und 4 h mit 60µM Isopropyl-β-D-Thiogalactopyranosid (IPTG) induziert. Durch Zugabe von 6 M Guanidinhydrochlorid wurde eine Lyse der Bakterien erreicht, anschließend wurde mit dem Lysat eine Chromatographie (Ni-NTA-Resin) in Gegenwart von 8 M Harnstoff entsprechend der Angaben des Herstellers (Diagen) des Chromatographie-Materials durchgeführt. Das gebundene Fusionsprotein wurde in einem Puffer mit pH 3,5 eluiert. Nach seiner Neutralisierung wurde das Fusionsprotein einer 18 % SDS-Polyacrylamid-Gelelektrophorese unterworfen und mit Coomassie-Blau angefärbt (vgl. Thomas, J.O. und Kornberg, R.D., J.Mol.Biol. 149 (1975), 709-733).

Es zeigte sich, daß ein erfindungsgemäßes (Fusions)protein in hochreiner Form hergestellt werden kann.

### Beispiel 2: Herstellung und Nachweis eines erfindungsgemäßen Antikörpers

Ein erfindungsgemäßes Fusionsprotein von Beispiel 1 wurde einer 18 % SDS-Polyacrylamid-Gelelektrophorese unterzogen. Nach Anfärbung des Gels mit 4 M Natriumacetat wurde eine ca. 260 kD Bande aus dem Gel herausgeschnitten und in Phosphat gepufferter Kochsalzlösung inkubiert. Gel-Stücke wurden sedimentiert, bevor die Proteinkonzentration des Überstandes durch eine SDS-Polyacrylamid-Gelelektrophorese, der eine Coomassie-Blau-Färbung folgte, bestimmt wurde. Mit dem Gel-gereinigten Fusionsprotein wurden Tiere wie folgt immunisiert:

### lmmunisierungsprotokoll für polyklonale Antikörper im Kaninchen

Pro Immunisierung wurden 35µg Gel-gereinigtes Fusionsprotein in 0,7 ml PBS und 0,7 ml komplettem bzw. inkomplettem Freund's Adjuvans eingesetzt.

| | |
|---|---|
| Tag 0 | 1. Immunisierung (komplettes Freund's Adjuvans) |
| Tag 14 | 2. Immunisierung (inkomplettes Freund's Adjuvans; icFA) |
| Tag 28 | 3. Immunisierung (icFA) |
| Tag 56 | 4. Immunisierung (icFA) |
| Tag 80 | Ausbluten |

Das Serum des Kaninchens wurde im Immunoblot getestet. Hierzu wurde ein erfindungsgemäßes Fusionsprotein von Beispiel 1 einer SDS-Polyacrylamid-Gelelektrophorese unterzogen und auf ein Nitrocellulosefilter übertragen (vgl. Khyse-Andersen, J., J. Biochem. Biophys. Meth. 10, (1984), 203-209). Die Western Blot-Analyse wurde wie in Bock, C.-T. et al., Virus Genes 8, (1994), 215-229, beschrieben, durchgeführt. Hierzu wurde das Nitrocellulosefilter eine Stunde bei 37°C mit einem ersten Antikörper inkubiert. Dieser Antikörper war das Serum des Kaninchens (1:10000 in PBS). Nach mehreren Waschschritten mit PBS wurde das Nitrocellulosefilter mit einem zweiten Antikörper inkubiert. Dieser Antikörper war ein mit alkalischer Phosphatase gekoppelter monoklonaler Ziege Anti-Kaninchen-IgG-Antikörper (Dianova) (1:5000) in PBS. Nach 30-minütiger Inkubation bei 37°C folgten mehrere Waschschritte mit PBS und anschließend die alkalische Phosphatase-Nachweisreaktion mit Entwicklerlösung (36µM 5' Bromo-4-chloro-3-indolylphosphat, 400µM Nitroblau-tetrazolium, 100mM Tris-HCl, pH 9.5, 100 mM NaCl, 5 mM MgCl₂) bei Raumtemperatur, bis Banden sichtbar waren.

Es zeigte sich, daß erfindungsgemäße, polyklonale Antikörper hergestellt werden können.

### Immunisierungsprotokoll für polyklonale Antikörper im Huhn

Pro Immunisierung wurden 40µg Gel-gereinigtes Fusionsprotein in 0,8 ml PBS und 0,8 ml komplettem bzw. inkomplettem Freund's Adjuvans eingesetzt.

| | |
|---|---|
| Tag 0 | 1. Immunisierung (komplettes Freund's Adjuvans) |
| Tag 28 | 2. Immunisierung (inkomplettes Freund's Adjuvans; icFA) |
| Tag 50 | 3. Immunisierung (icFA) |

Aus Eigelb wurden Antikörper extrahiert und im Western Blot getestet. Es wurden erfindungsgemäße, polyklonale Antikörper nachgewiesen.

### Immunisierungsprotokoll für monoklonale Antikörper der Maus

Pro Immunisierung wurden 12µg Gel-gereinigtes Fusionsprotein in 0,25 ml PBS und 0,25 ml komplettem bzw. inkomplettem Freund's Adjuvans eingesetzt; bei der 4. Immunisierung war das Fusionsprotein in 0,5 ml (ohne Adjuvans) gelöst.

| | |
|---|---|
| Tag 0 | 1. Immunisierung (komplettes Freund's Adjuvans) |
| Tag 28 | 2. Immunisierung (inkomplettes Freund's Adjuvans; icFA) |
| Tag 56 | 3. Immunisierung (icFA) |
| Tag 84 | 4. Immunisierung (PBS) |
| Tag 87 | Fusion |

Überstände von Hybridomen wurden im Western Blot getestet. Erfindungsgemäße, monoklonale Antikörper wurden nachgewiesen.

## Patentansprüche

1. SRCR Domäne-enthaltendes Protein, wobei das Protein zumindest die Aminosäuresequenz von Fig. 1 oder eine hiervon durch eine oder mehrere Aminosäuren unterschiedliche Aminosäuresequenz umfaßt, wobei das Protein mit letzterer Aminosäuresequenz auf der DNA-Ebene mit der DNA von Fig. 1 bei 20°C unter dem Schmelzpunkt der DNA hybridisiert.

2. SRCR Domäne-enthaltendes Protein nach Anspruch 1, wobei das Protein die Aminosäuresequenz von Fig. 2 oder eine hiervon durch eine oder mehrere Aminosäuren unterschiedliche Aminosäuresequenz aufweist, wobei das Protein mit letzterer Aminosäuresequenz auf der DNA-Ebene mit der DNA von Fig. 2 bei 20°C unter dem Schmelzpunkt der DNA hybridisiert.

3. Nukleinsäure, kodierend für das Protein nach Anspruch 1.

4. Nukleinsäure, kodierend für das Protein nach Anspruch 2.

5. Nukleinsäure nach Anspruch 3 oder 4, wobei die Nukleinsäure eine DNA ist.

6. DNA nach Anspruch 5, wobei die DNA umfaßt:
(a) die DNA von Fig. 1 oder 2 oder eine hiervon durch ein oder mehrere Basenpaare unterschiedliche DNA, wobei letztere DNA mit der DNA von Fig. 1 oder 2 bei 20°C unter dem Schmelzpunkt der DNA hybridisiert, oder
(b) eine mit der DNA von (a) über den degenerierten genetischen Code verwandte DNA.

7. Vektor, umfassend die DNA nach Anspruch 5 oder 6.

8. Vektor nach Anspruch 7, wobei der Vektor ein Expressionsvektor ist.

9. Transformante, enthaltend den Vektor nach Anspruch 7.

10. Transformante, enthaltend den Expressionsvektor nach Anspruch 8.

11. Verfahren zur Herstellung des Proteins nach Anspruch 1 oder 2, umfassend die Kultivierung der Transformante nach Anspruch 10 unter geeigneten Bedingungen.

12. Antikörper, gerichtet gegen das Protein nach Anspruch 1 oder 2.

13. Verwendung des Proteins nach Anspruch 1 oder 2 zur Herstellung eines Reagens zur Diagnose und/oder Therapie einer Tumorerkrankung.

14. Verwendung der Nukleinsäure nach einem der Ansprüche 3-6 zur Herstellung eines Reagens zur Diagnose und/oder Therapie einer Tumorerkrankung.

15. Verwendung nach Anspruch 13 oder 14, wobei die Tumorerkrankung das zentrale Nervensystem oder die Brust betrifft.

16. Verwendung nach Anspruch 15, wobei die Tumorerkrankung ein Medulloblastom, ein Glioblastom oder Brustkrebs ist.

## Claims

1. A protein containing an SRCR domain, wherein the protein comprises at least the amino acid sequence of fig. 1 or an amino acid sequence differing therefrom by one or several amino acids, wherein on the DNA level the protein having the latter amino acid sequence hybridizes with the DNA of fig. 1 at 20°C below the melting point of the DNA.

2. The protein containing an SRCR domain according to claim 1, wherein the protein includes the amino acid sequence of fig. 2 or an amino acid sequence differing therefrom by one or several amino acids, wherein on the DNA level the protein having the latter amino acid sequence hybridizes with the DNA of fig. 2 at 20°C below the melting point of the DNA.

3. A nucleic acid coding for the protein according to claim 1.

4. The nucleic acid coding for the protein according to claim 2.

5. The nucleic acid according to claim 3 or 4, wherein the nucleic acid is a DNA.

6. The DNA according to claim 5, wherein the DNA comprises:
(a) the DNA of fig. 1 or 2 or a DNA differing therefrom by one or several base pairs, the latter DNA hybridizing with the DNA of fig. 1 or 2 at 20°C below the melting point of the DNA, or
(b) a DNA related to the DNA from (a) via the degenerated genetic code.

7. A vector comprising the DNA according to claim 5 or 6.

8. The vector according to claim 7, wherein the vector is an expression vector.

9. A transformant containing the vector according to claim 7.

10. The transformant containing the expression vector according to claim 8.

11. A method of producing the protein according to claim 1 or 2, comprising the culturing of the transformant according to claim 10 under suitable conditions.

12. Antibodies directed against the protein according to claim 1 or 2.

13. Use of the protein according to claim 1 or 2 for preparing a reagent for diagnosing and/or treating a tumoral disease.

14. Use of the nucleic acid according to any one of claims 3 to 6 for preparing a reagent for diagnosing and/or treating a tumoral disease.

15. Use according to claim 13 or 14, wherein the tumoral disease affects the central nervous system or the breast.

16. Use according to claim 15, wherein the tumoral disease is a medulloblastoma, glioblastoma or breast cancer.

## Revendications

1. Une protéine contenant un domaine SRCR, ladite protéine comprenant au moins la séquence d'acide aminé de la figure 1 ou une séquence d'acide aminé en différant par un ou plusieurs acides aminés, dans laquelle la protéine avec cette dernière séquence d'acide aminé s'hybridise au niveau de l'ADN avec l'ADN de la figure 1, à 20°C en dessous du point de fusion de l'ADN.

2. Une protéine contenant un domaine SRCR, selon la revendication 1, ladite protéine comprenant au moins la séquence d'acide aminé de la figure 2 ou une séquence d'acide aminé en différant par un ou plusieurs acides aminés, dans laquelle la protéine avec cette dernière séquence d'acide aminé s'hybridise au niveau de l'ADN avec l'ADN de la figure 2, à 20°C en dessous du point de fusion de l'ADN.

3. Acide nucléique codant pour la protéine de la figure 1.

4. Acide nucléique codant pour la protéine de la figure 2.

5. Acide nucléique selon la revendication 3 ou 4, **caractérisée en ce que** l'acide nucléique est une ADN.

6. ADN selon la revendication 5, dans laquelle cette ADN contient:
a) l'ADN de la figure 1 ou de la figure 2 ou une ADN se distinguant de ces dernières par une ou plusieurs paire de bases, cette dernière étant hybridisée avec l'ADN de la figure 1 ou de la figure 2, à 20°C en dessous du point de fusion de l'ADN.
b) une ADN apparentée ayant le code génétique dégénéré de l'ADN de a).

7. Vecteur contenant l'ADN selon la revendication 5 ou 6.

8. Vecteur selon la revendication 7, ledit vecteur étant un vecteur d'expression.

9. Tranformant contenant le vecteur selon la revendication 7.

10. Tranformant contenant le vecteur d'expression selon la revendication 8.

11. Procédé de préparation de la protéine selon la revendication 1 ou 2, impliquant la culture du transformant de la revendication 10 dans des conditions appropriées.

12. Anticorps dirigé contre la protéine selon la revendication 1 ou 2.

13. Application de la protéine selon la revendication 1 ou 2 pour la préparation d'un réactif pour le diagnostic et/ou la thérapie d'une maladie tumorale.

14. Application de l'acide nucléique selon une des revendications 3 à 6 pour la préparation d'un réactif pour le diagnostic et/ou la thérapie d'une maladie tumorale.

15. Application selon la revendication 13 ou 14, dans laquelle la maladie tumorale affecte le système nerveux ou le sein.

16. Application selon la revendication 15, dans laquelle la maladie tumorale est un blastome médullaire, un glioblastome ou un cancer du sein.
